# Europäisches Patentamt
# European Patent Office
# Office européen des brevets

(19)

(11) Veröffentlichungsnummer : **0 379 971 B1**

(12)

# EUROPÄISCHE PATENTSCHRIFT

(45) Veröffentlichungstag der Patentschrift :
**24.03.93 Patentblatt 93/12**

(51) Int. Cl.$^5$ : **C07C 53/124,** C07C 51/14

(21) Anmeldenummer : **90100988.6**

(22) Anmeldetag : **18.01.90**

(54) **Kontinuierliches Verfahren zur Herstellung von Isobuttersäure.**

(30) Priorität : **25.01.89 DE 3902104**

(43) Veröffentlichungstag der Anmeldung :
**01.08.90 Patentblatt 90/31**

(45) Bekanntmachung des Hinweises auf die
Patenterteilung :
**24.03.93 Patentblatt 93/12**

(84) Benannte Vertragsstaaten :
**BE DE DK ES FR GB IT**

(56) Entgegenhaltungen :
**EP-A- 0 031 886**
**EP-A- 0 079 497**

(73) Patentinhaber : **Röhm GmbH**
**Kirschenallee Postfach 4242**
**W-6100 Darmstadt 1 (DE)**

(72) Erfinder : **Ruppert, Wolfgang, Dr.-Ing.**
**Am Mühlgraben 9**
**W-6101 Bickenbach (DE)**
Erfinder : **Plösser, Willi**
**Eberstädter Strasse 23**
**W-6104 Seeheim-Jugenheim 3 (DE)**

Jouve, 18, rue Saint-Denis, 75001 PARIS

## Beschreibung

Die Erfindung betrifft ein Verfahren zur kontinuierlichen Herstellung von Isobuttersäure durch Koch'sche Synthese aus Propen, Kohlenmonoxid, Wasser in Fluorwasserstoff als Koch'schem Katalysator.

Stand der Technik

Aus der DE-C 30 33 655 ist ein Verfahren der oben genannten Art bekannt, bei dem die Ausgangsstoffe jeweils über getrennte Leitungen in den Reaktor eingeführt werden. Das Molverhältnis der gegebenenfalls gemischt zugeführten Mengen an Propen und Kohlenmonoxid beträgt 1 : 1,5. Ein Einfluß dieses Mischungsverhältnisses auf die Ausbeute ist nicht zu erkennen. Bei einer Verweilzeit von 5 Minuten wird in einem Labor-Druckreaktor bei 120°C und 120 bar CO-Druck und einem Zuführungsverhältnis von 10 Mol Fluorwasserstoff pro Mol Propen eine Ausbeute von 91 bis 92 % an Isobuttersäure erreicht. Führt man die Umsetzung unter gleichen Bedingungen in einem Technikums-Reaktor durch, so sinkt die Ausbeute auf 88 bis 89 %.

Aufgabe und Lösung

Die Ausbeute an Isobuttersäure war unter Einhaltung der übrigen Reaktionsbedingungen zu erhöhen. Es wurde gefunden, daß die Ausbeute steigt, wenn das frisch zugeführte Propen mit mehr als der 1,5-fach molaren Menge an Kohlenmonoxid vermischt und in den Reaktor eingeführt wird. In dem vorerwähnten Technikums-Reaktor wurde durch diese Maßnahme bei einem Molverhältnis Propen : CO von 1 : 4 eine Steigerung der Ausbeute auf 94 % erreicht.

Ausführung der Erfindung

Das günstigste Verhältnis von Propen zu Kohlenmonoxid liegt zwischen 1 : 1,8 bis 1 : 15, insbesondere bis 1 : 10. Bei einer weiteren Steigerung des Kohlenmonoxidgehaltes im Gaszustrom wird keine Verbesserung der Ausbeute mehr erreicht, jedoch nimmt die Wirtschaftlichkeit ab. Bei der Umsetzung der Ausgangsstoffe wird Kohlenmonoxid höchstens in äquimolarem Verhältnis zum Propen verbraucht, so daß kontinuierlich ein Teil der Gasphase mit dem überschüssigen Kohlenmonoxid aus dem Reaktor abgeleitet werden muß. Das Verfahren ist nur dann wirtschaftlich zu betreiben, wenn das nicht verbrauchte Kohlenmonoxid komprimiert und in den Prozeß zurückgeführt wird.

Die abgenommene Gasphase besteht zunächst aus einem Gemisch von Kohlenmonoxid und Fluorwasserstoff sowie gegebenenfalls kleinen Mengen von Propan, Wasserstoff und gasförmigen Nebenprodukten. Wird das Gasgemisch insgesamt in den Prozeß zurückgeführt, so reichern sich die genannten Verunreinigungen in der Gasphase an und werden deshalb durch laufende Abtrennung eines Teils der Gasphase ausgeschleust.

Aus wirtschaftlichen Gründen erscheint es zunächst zwar sinnvoll, das rückgeführte Kohlenmonoxid ohne vorherige Reinigung von Fluorwasserstoff unter Ersatz des verbrauchten Kohlenmonoxids mit dem zuzuführenden Propen zu vermischen, weil es beim Einleiten in den Reaktor ohnehin wieder mit Fluorwasserstoff vermischt wird. Es hat sich jedoch gezeigt, daß beim Vermischen des Propens mit dem durch Fluorwasserstoff verunreinigten Kohlenmonoxid eine Polymerisation des Propens in der Mischkammer eintritt. Der dadurch bedingte Verlust an Propen ist so groß, daß die Ausbeute an Isobuttersäure auf weniger als 70 % sinkt.

Es wurde festgestellt, daß die Polymerisation des Propens durch den Fluorwasserstoff-Gehalt des Gasgemisches ausgelöst wird. Erst bei einem Gehalt an Fluorwasserstoff unter 5 Mol-%, vorzugsweise unter 3 Mol-% in dem Gasgemisch sinkt die Polymerisation auf eine vernachlässigbares Ausmaß ab. Vorzugsweise sollte der HF-Gehalt nicht mehr als 1 Mol-% betragen.

Bei einer bevorzugten Ausführungsform der Erfindung wird daher des rückgeführte Kohlenmonoxid von Fluorwasserstoff gereinigt. Zweckmäßig wird es durch Waschen mit einer Absorptionsflüssigkeit von Fluorwasserstoff befreit. Als Absorptionsflüssigkeit kann Wasser verwendet werden, was jedoch wegen der hohen Korrosivität der entstehenden Flußsäure manchmal nachteilig ist. Vorzugsweise wird Isobuttersäure als Absorptionsflüssigkeit verwendet, da sie ein hohes Bindungsvermögen für Fluorwasserstoff hat. Das bei der Absorption entstehende Gemisch ist wenig korrosiv und kann zusammen mit dem Reaktorabstrom aufgearbeitet werden. Es ist nicht erforderlich, reine Isobuttersäure zur Absorption einzusetzen. Man kann beispielsweise den Sumpfabstrom der Destillation, bei der Fluorwasserstoff aus dem flüssigen Reaktionsgemisch abdestilliert wird, verwenden. Er enthält neben etwa 80 bis 99 Gew.-% Isobuttersäure die bei der Reaktion entstandenen Hochsieder.

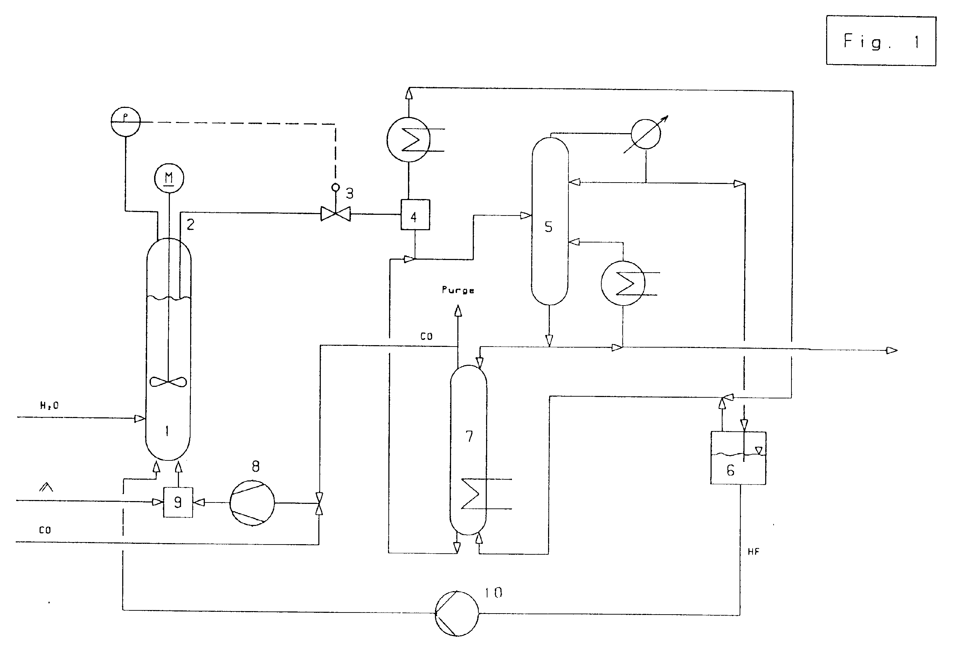

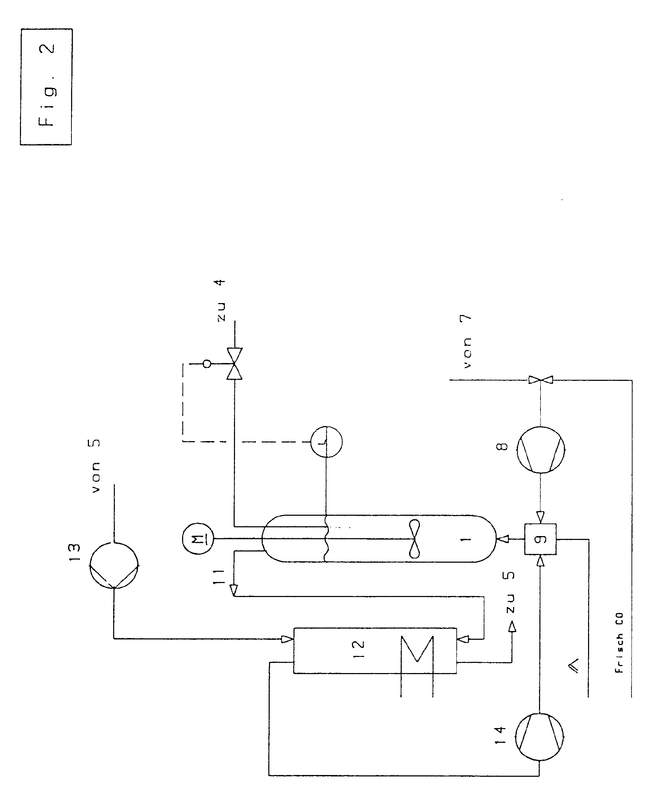

Zweckmäßige Ausgestaltungen des Verfahrens werden in den Figuren 1 und 2 durch Fließbilder erläutert.

**Figur 1** stellt eine Verfahrensvariante dar, bei der die vom Reaktor abgenommene Gasphase entspannt, gereinigt, komprimiert und dann mit Propen unter Druck vermischt wird.

**Figur 2** betrifft eine Verfahrensvariante, bei der der Hauptteil der Gasphase unter Druck gereinigt und ohne Entspannung mit Propen vermischt und in den Reaktor zurückgeführt wird.

Aus dem Reaktor 1 der in Figur 1 dargestellten Anlage wird über das Rohr 2 flüssiges und gasförmiges Reaktionsgemisch entnommen, so daß sich das Flüssigkeitsniveau auf das untere Ende des Rohres 2 einstellt. Am Entspannungsventil 3 wird der Druck des abgenommenen Reaktionsgemisches von etwa 50 bis 140, vorzugsweise 100 bis 140 bar auf etwa 1 bis 5 bar vermindert. Man kann auch in mehreren Druckstufen entspannen und jeweils einen Teil der Gasphase abtrennen. Im Abscheider 4 werden die gasförmigen Anteile abgetrennt. Die flüssigen Anteile werden in der Destillationskolonne 5 in ein überwiegend aus Fluorwasserstoff bestehendes Destillat und einen Sumpfabstrom, der aus 80 - 99 Gew.-% Isobuttersäure und zum restlichen Teil aus Hochsiedern besteht, getrennt. Das Destillat wird in den Zwischenbehälter 6 geleitet, wo sich restliches Kohlenmonoxid vom flüssigen Fluorwasserstoff trennt. Die aus den Behältern 4 und 6 entnommenen Gasphasen werden vereinigt und in den Gegenstromwäscher 7 geleitet, wo mit einem Teilstrom des Sumpfabstroms der Kolonne 5 der Fluorwasserstoff ausgewaschen wird. Die Absorptionswärme kann durch Kühlung abgeführt werden. Die Menge der Waschflüssigkeit sollte so bemessen werden, daß der HF-Gehalt in der ablaufenden Waschflüssigkeit im Bereich von 1 bis 50 Gew.-% liegt. Wenn beispielsweise die Gasphase zu 85 % aus Kohlenmonoxid und zu 15 % aus Fluorwasserstoff besteht, werden 2 bis 95 % des gesamten Sumpfabstroms in den Wascher 7 geleitet. Das aus dem Wascher 7 abströmende Gas hat nur noch einen HF-Gehalt von 0,5 Gew.-%.

Von der gereinigten Gasphase wird ein Teil, z.B. 2 %, abgetrennt, um Verunreinigungen auszuschleusen. Der Hauptteil der Gasphase wird mit dem Frisch-Kohlenmonoxid vereinigt und im Kompressor 8 auf den Reaktionsdruck von 50 bis 140, vorzugsweise 100 bis 140 bar verdichtet und in der Mischkammer 9 mit Frisch-Propen vermischt. Um den Gaszustand aufrechtzuerhalten, wird die Temperatur der Mischung auf mindestens 70°C eingestellt. Das Gasgemisch wird dann in den Reaktor 1 eingeleitet. Gleichzeitig wird aus dem Behälter 6 flüssiger Fluorwasserstoff über die Pumpe 10 in den Reaktor geleitet.

Bei der in Figur 2 dargestellten Verfahrensvariante wird die Hauptmenge der Gasphase über die Leitung 11 in den Druckwäscher 12 geleitet, wo sie im Gegenstrom mit der Waschflüssigkeit von Fluorwasserstoff gereinigt und mittels des Gebläses 14 in die Mischkammer 9 geleitet wird. Wenn als Reaktor 1 ein Treibstrahlreaktor eingesetzt wird, kann der CO-Gaskreislauf über eine oder mehrere Zweistoffdüsen angetrieben werden. In diesem Fall ist das Gebläse 14 entbehrlich. Die Waschflüssigkeit wird mittels der Pumpe 13 auf den Betriebsdruck von 50 bis 140 bar gebracht und am Kopf des Wäschers 12 eingeleitet. Wird der Sumpfabstrom der Kolonne 5 als Waschflüssigkeit verwendet, so wird die mit Fluorwasserstoff beladene Waschflüssigkeit nach Entspannung in die Kolonne 5 zurückgeleitet. Der übrige Teil der Anlage entspricht dem Schema der Figur 1. Die in Figur 2 dargestellte Variante ist besonders bei einem hohen Verhältnis von Kohlenmonoxid zu Propen, beispielsweise über 4 : 1, vorteilhaft.

Anstelle der Extraktion mittels einer Waschflüssigkeit kann bei hohen Drucken, beispielsweise über 100 bar, der Fluorwasserstoff durch Kühlung kondensiert und sein Anteil in der Gasphase dadurch ausreichend erniedrigt werden. Der Wäscher 12 wird in diesem Falle durch einen Kondensator ersetzt. Wenn beispielsweise der Gehalt an Fluorwasserstoff in dem durch die Leitung 11 strömenden Gas 15 % beträgt, kann durch Abkühlung auf 20 bis 30°C der HF-Gehalt im Gas auf 0,8 Gew.-% gesenkt werden.

BEISPIEL 1

In einem Rührautoklav mit 1,6 l Rauminhalt werden kontinuierlich Propen, Kohlenmonoxid, Wasser und Fluorwasserstoff im Molverhältnis 1 : 1,3 - 2,8 : 0,8 - 1,0 : 10 bei 120 Grad C, einem Druck von 120 bar und einer Rührgeschwindigkeit von 1 500 Upm umgesetzt. Kohlenmonoxid und HF-freies Propen werden vor dem Einspeisen in den Autoklav gemischt. In Abhängigkeit von der Zusammensetzung dieses Gasgemisches werden folgende Ausbeuten (in % d.Th. bez. auf Propen) erzielt:

| Versuch Nr. | Molverhältnis Propen : CO | Ausbeute Isobuttersäure | Oligomeren Bildung (%) |
|---|---|---|---|
| 1 (Vergleich) | 1 : 1,3 | 81,5 % | 5 % |
| 2 | 1 : 1,9 | 90,5 % | 6 % |
| 3 | 1 : 2,8 | 93,5 % | 5 % |

BEISPIEL 2

In einer Vorrichtung wie in Beispiel 1 wird ein Gemisch von Propen, CO, Wasser und HF im Molverhältnis 1 : 3,4 : 1 : 9,6 bei 120°C, 120 bar, 1500 Upm kontinuierlich umgesetzt. Das eingespeiste CO enthielt 0,5 - 1,0 Mol-% HF.
Bei einem Vergleichsversuch bei dem das Verhältnis Propen : CO = 1 : 5,9 betrug enthielt das CO 20 % HF. Ausbeuten:

| HF-Anteil in CO(Mol%) | Molverhältnis Propen : CO | Verweilzeit | Ausbeute |
|---|---|---|---|
| 0,5 - 1 | 1 : 3,4 | 4,6 min | 89,6 % |
| 20 | 1 : 5,9 | 5,7 min | 67,4 % |

Obwohl im letztgenannten Versuch der CO-Gehalt höher war, was nach Beispiel 1 vorteilhaft sein sollte, lag die Ausbeute niedriger. Dies ist auf den höheren HF-Gehalt in CO zurückzuführen.

**Patentansprüche**

1.  Verfahren zur kontinuierlichen Herstellung von Isobuttersäure durch Koch'sche Synthese aus Propen, Kohlenmonoxid, Wasser in Gegenwart von Fluorwasserstoff als Koch'schem Katalysator unter Druck in einem Druckreaktor bei kontinuierlicher Zufuhr von flüssigen und gasförmigen Ausgangsstoffen, die eine Gasmischung von Propen und Kohlenmonoxid enthalten können, und kontinuierlicher Abnahme von flüssigem und/oder gasförmigem Reaktionsgemisch, Entspannung des abgenommenen Reaktionsgemisches und Rückführung des Fluorwasserstoffs und des nicht verbrauchten Kohlenmonoxids in den Reaktor,
    dadurch gekennzeichnet,
    daß das frisch zugeführte Propen mit mehr als der 1,5-fach molaren Menge an Kohlenmonoxid vermischt und in den Reaktor eingeführt wird.

2.  Verfahren nach Anspruch 1, dadurch gekennzeichnet, daß das Propen mit Kohlenmonoxid mit einem Fluorwasserstoff-Gehalt unter 5 Mol-% vermischt wird.

3.  Verfahren nach Anspruch 2, dadurch gekennzeichnet, daß das rückgeführte Kohlenmonoxid von Fluorwasserstoff befreit und danach mit Propen vermischt wird.

4.  Verfahren nach Anspruch 3, dadurch gekennzeichnet, daß das Kohlenmonoxid durch Waschen mit einer Absorptionsflüssigkeit von Fluorwasserstoff befreit wird.

5.  Verfahren nach Anspruch 4, dadurch gekennzeichnet, daß als Absorptionsflüssigkeit Wasser verwendet wird.

**6.** Verfahren nach Anspruch 4, dadurch gekennzeichnet, daß als Absorptionsflüssigkeit Isobuttersäure verwendet wird.

**7.** Verfahren nach Anspruch 6, dadurch gekennzeichnet, daß als Isobuttersäure der Sumpfabstrom der Destillation, bei der Fluorwasserstoff aus dem flüssigen Reaktionsgemisch abdestilliert wird, eingesetzt wird.

**8.** Verfahren nach einem oder mehreren der Ansprüche 1 bis 7, dadurch gekennzeichnet, daß das Verhältnis von Kohlenmonoxid zu Propen zwischen 1,8 : 1 und 15 : 1 liegt.

## Claims

**1.** A process for the continuous preparation of isobutyric acid by Koch's synthesis from propene, carbon monoxide, water, in the presence of hydrogen fluoride as Koch's catalyst, under pressure in a pressure reactor, consisting of the continuous supply of liquid and gaseous starting materials which may contain a gaseous mixture of propene and carbon monoxide, and continuous discharge of liquid and/or gaseous reaction mixture, expansion of the reaction mixture removed and returning the hydrogen fluoride and the carbon monoxide, not used, into the reactor, characterised in that the freshly supplied propene is mixed with a quantity of more than 1.5 times the molar amount of carbon monoxide and introduced into the reactor.

**2.** A process according to claim 1, characterised in that the propene is mixed with carbon monoxide having a hydrogen fluoride content of less than 5 mol %.

**3.** A process according to claim 2, characterised in that the returned carbon monoxide is freed of hydrogen fluoride and then mixed with propene.

**4.** A process according to claim 3, characterised in that the carbon monoxide is freed of hydrogen fluoride by washing with an absorption liquid.

**5.** A process according to claim 4, characterised in that water is used as absorption liquid.

**6.** A process according to claim 4, characterised in that isobutyric acid is used as absorption liquid.

**7.** A process according to claim 6, characterised in that the discharge from the bottom region of the distillation, in which hydrogen fluoride is distilled off from the reaction mixture, is used as isobutyric acid.

**8.** A process according to one or more of claims 1 to 7, characterised in that the ratio of carbon monoxide to propene lies in the range between 1.8 : 1 and 15 : 1.

## Revendications

**1.** Procédé de préparation continue d'acide isobutyrique par synthèse de Koch à partir de propylène, de monoxyde de carbone et d'eau, en présence d'acide fluorhydrique servant de catalyseur de Koch, sous pression dans un réacteur à pression, avec apport continu de substances de départ liquides et gazeuses, qui peuvent contenir un mélange gazeux de propylène et de monoxyde de carbone, et avec extraction continue de mélange réactionnel liquide et/ou gazeux, détente du mélange réactionnel extrait et renvoi de l'acide fluorhydrique et du monoxyde de carbone non consommé dans le réacteur, caractérisé en ce que le propylène nouvellement introduit est mélangé à plus de 1,5 fois sa quantité molaire de monoxyde de carbone et est envoyé dans le réacteur.

**2.** Procédé selon la revendication 1, caractérisé en ce que le propylène est mélangé à du monoxyde de carbone ayant une teneur en acide fluorhydrique inférieure à 5% en moles.

**3.** Procédé selon la revendication 2, caractérisé en ce que le monoxyde de carbone renvoyé est débarrassé de l'acide fluorhydrique, puis est mélangé au propylène.

**4.** Procédé selon la revendication 3, caractérisé en ce que le monoxyde de carbone est débarrassé de l'acide fluorhydrique par lavage avec un liquide absorbant.

5. Procédé selon la revendication 4, caractérisé en ce qu'on utilise de l'eau comme liquide absorbant.

6. Procédé selon la revendication 4, caractérisé en ce qu'on utilise de l'acide isobutyrique comme liquide absorbant.

7. Procédé selon la revendication 6, caractérisé en ce qu'on utilise, comme acide isobutyrique, l'effluent de bas de colonne de la distillation par laquelle l'acide fluorhydrique est chassé du mélange réactionnel liquide.

8. Procédé selon l'une quelconque des revendications 1 à 7, caractérisé en ce que le rapport du monoxyde de carbone au propylène se situe entre 1,8 : 1 et 15 : 1.

Fig. 1

Fig. 2

EP 0 379 971 B1